# EUROPÄISCHE PATENTSCHRIFT

(11) **EP 1 250 102 B1**
(45) Veröffentlichungstag und Bekanntmachung des Hinweises auf die Patenterteilung: **18.06.2003**
(21) Anmeldenummer: 00978976.9
(22) Anmeldetag: 09.11.2000
(51) Int. Cl.: A61B 18/14

(54) **BIPOLARES FASSINSTRUMENT**
BIPOLAR GRIPPING DEVICE
INSTRUMENT DE PINCAGE BIPOLAIRE

(30) Priorität: 25.01.2000 DE 10003020
(43) Veröffentlichungstag der Anmeldung: 23.10.2002
(73) Patentinhaber: Aesculap AG & Co. KG, 78532 Tuttlingen (DE)
(72) Erfinder: LUTZE, Theodor, 78582 Balgheim (DE); MAYENBERGER, Rupert, 78239 Rielasingen (DE); ROTHWEILER, Chritstoph, 78166 Donaueschingen (DE); WEISSHAUPT, Dieter, 78194 Immendingen (DE)
(74) Vertreter: Böhme, Ulrich, Dr. Dipl.-Phys.
(86) Internationale Anmeldenummer: EP0011052
(87) Internationale Veröffentlichungsnummer: WO01054604

(56) Entgegenhaltungen:
- EP-A- 0 598 348
- WO-A-96/05776
- WO-A-99/40861
- DE-U- 20 001 204
- US-A- 5 797 941
- US-A- 5 984 939

## Beschreibung

Die Erfindung betrifft ein bipolares Faßinstrument mit zwei gegeneinander bewegbaren, elektrisch voneinander isolierten und jeweils mit einem Pol einer elektrischen Hochfrequenzspannungsquelle verbindbaren Klemmbacken, die jeweils zwei im Abstand zueinander angeordnete, zwischen sich einen Längsschlitz ausbildende Klemmelemente mit je einer Klemmfläche aufweisen, und mit einer Schneideinrichtung, die ein in den Längsschlitzen der Klemmbacken verschiebbares Schneidelement mit einer Schneide umfaßt, wobei das Schneidelement im Längsschlitz des einen Klemmbackens angeordnet ist.

Mit bipolaren Faßinstrumenten ist es möglich, Gewebe im gefaßten Bereich zu koagulieren, so daß in diesem Bereich Blutungen gestillt werden können.

Es ist bekannt, mit derartigen Instrumenten beispielsweise bei Gefäßen bestimmte Bereiche zu koagulieren und dann in diesem koagulierten Bereich die Gefäße durch einen Schnitt zu durchtrennen, wobei die Gefahr von Blutungen durch die vorangehende Koagulation minimiert wird. Dazu sind zwei völlig getrennte Verfahrensschritte notwendig, die üblicherweise auch mit unterschiedlichen Instrumenten ausgeführt werden, nämlich das Koagulieren mit einem bipolaren Faßinstrument einerseits und das Durchtrennen des koagulierten Gewebes mittels einer Schneideinrichtung andererseits.

Es sind auch bipolare Faßinstrumente bekannt, in die eine Schneideinrichtung integriert ist (Firmenprospekt CIRCON, TRIPOLAR Cutting Forceps; US-Patent 5 458 598). Bei diesem Instrument sind die Klemmbacken des Faßinstrumentes durch Längsschlitze in zwei nebeneinander angeordnete Klemmflächen unterteilt, und in die Längsschlitze kann in Längsrichtung des Instrumentes ein mit einer Schneide versehenes Schneidelement eingeschoben werden, welches zwischen den Klemmflächen gehaltene Gewebeteile nach dem Koagulieren durchtrennt. Bei diesem vorbekannten Instrument müssen für das Schließen der Klemmbacken zum Zwecke des Koagulierens und zum Vorschieben des Schneidelements der Schneideinrichtung unterschiedliche Antriebsmechanismen vorgesehen sein, dieses Instrument, das vorzugsweise als Rohrschaftinstrument ausgebildet ist, ist somit relativ kompliziert ausgebildet, außerdem muß der Benutzer dieses Instrumentes zwei getrennte Antriebsmechanismen bedienen, um nacheinander Gewebe zu erfassen und es dann zu durchtrennen.

In der WO 96/05776 ist ein bipolares Faßinstrument beschrieben, bei dem eine Schneide in einem Längsschlitz zwischen zwei Klemmelementen eines Klemmbackens angeordnet ist, diese Schneide kann nach erfolgter Koagulation so verschoben werden, daß das zwischen den Klemmbacken gehaltene Material durchgeschnitten wird. Wie das Schneidelement zum Durchtrennen des Gewebes bewegt werden soll, wird in dieser Druckschrift nicht näher erläutert, es ist aber davon auszugehen, daß dazu eine bewußte manuelle Verschiebung des Schneidelementes oder eine Verschiebung mit einem getrennten Antriebsmechanismus notwendig ist.

Es ist Aufgabe der Erfindung, ein bipolares Faßinstrument der gattungsgemäßen Art so auszubilden, daß die Bedienung vereinfacht wird, insbesondere dadurch, daß der Benutzer nur noch einen Betätigungsmechanismus benötigt, um nacheinander Gewebe zu erfassen und dann zu durchtrennen.

Diese Aufgabe wird bei einem bipolaren Faßinstrument der eingangs beschriebenen Art.erfindungsgemäß dadurch gelöst, daß die Schneide im Längsschlitz zwischen den Klemmelementen dieses Klemmbackens aufgenommen ist und nicht über dessen Klemmflächen in Richtung auf den anderen Klemmbacken übersteht, und daß die Klemmelemente dieses Klemmbackens relativ zu dem Schneidelement entgegen der Schließbewegung der Klemmbacken elastisch so weit bewegbar sind, daß die Schneide des Schneidelements über die Klemmflächen hervorsteht.

Beim Beginn der Schließbewegung der Klemmbacken ist also das Schneidelement im Längsschlitz des einen Klemmbackens so aufgenommen, daß die Schneide des Schneidelementes sich im Inneren des Längsschlitzes befindet und somit nicht wirksam wird. Das Instrument wirkt in diesem Falle wie ein konventionelles bipolares Faßinstrument, bei dem die Klemmflächen an das zu ergreifende Gewebe angelegt werden, ohne daß das Schneidelement überhaupt in Erscheinung tritt. In diesem Teil der Schließbewegung kann in herkömmlicher Weise durch Anlegen einer Hochfrequenzspannung koaguliert werden, das bipolare Faßinstrument kann nach dem Koagulieren wie ein konventionelles bipolares Faßinstrument auch wieder entfernt werden.

Wenn die Schließbewegung bei diesem bipolaren Faßinstrument dagegen nach dem Koagulieren fortgeführt wird, werden die Klemmbacken so kräftig gegeneinander bewegt, daß die Klemmelemente des Klemmbackens, der die Schneideinrichtung trägt, elastisch entgegen der Schließbewegung der Klemmbacken bewegt werden, und dies gibt die Schneide der Schneideinrichtung frei, die dann durch die elastische Verschiebung der Klemmelemente über die Klemmfläche hervorsteht. Das Instrument ist daher jetzt ein Schneidinstrument, bei dem das Schneidelement unter Durchtrennung des zwischen den Klemmbakken angeordneten Gewebes bis in den Längsschlitz des anderen Klemmbackens gelangen kann. Diese Schneidbewegung schließt sich unmittelbar an die normale Schließbewegung der Klemmbacken an, der Benutzer muß nur einen Antriebsmechanismus betätigen, bei leichter Betätigung erfolgt lediglich ein Klemmen und Festhalten des zu koagulierenden Gewebes, bei kräftigem Schließen der Klemmbacken dagegen erfolgt zusätzlich ein Durchtrennen der zwischen den Klemmbacken gehaltenen Gewebeteile.

Bei einer ersten bevorzugten Ausführungsform ist vorgesehen, daß sowohl das Schneidelement als auch die Klemmelemente einstückig mit dem Klemmbacken ausgebildet sind und daß das Schneidelement ein starres Teil des Klemmbackens ist, während die Klemmelemente über elastisch verformbare Bereiche mit den übrigen Teilen des Klemmbackens verbunden sind. Insbesondere können die elastisch verformbaren Bereiche durch elastisch verbiegbare Stege mit geringer Höhe gebildet werden.

Bei einer anderen Ausführungsform ist vorgesehen, daß der Klemmbacken zweiteilig ausgebildet ist mit einem starren ersten Teil, welches das Schneidelement und die Lagerung des Klemmbackens am Faßinstrument umfaßt, und mit einem zweiten Teil, welches die Klemmelemente umfaßt und welches am ersten Teil elastisch bewegbar gelagert ist.

Beispielsweise können die Klemmelemente elastisch verschwenkbar'an dem ersten Teil gelagert sein.

Es ist günstig, wenn das zweite Teil U-förmig ausgebildet ist mit zwei parallelen, die Klemmelemente bildenden Schenkeln und einem diese verbindenden, den Längsschlitz zum distalen Ende des Klemmbackens hin abschließenden Steg und wenn das zweite Teil an den freien Enden der Schenkel drehbar an dem ersten Teil gelagert ist. Es ergibt sich dadurch eine sehr stabile Anordnung des die Klemmflächen tragenden zweiten Teils des Klemmbackens.

Der Längsschlitz in dem anderen Klemmbacken, der keine Schneideinrichtung aufweist, ist vorzugsweise an beiden Enden geschlossen, so daß die Schneideinrichtung beim Schneidvorgang in diesen allseits geschlossenen Längsschlitz eintaucht, auch dies trägt dazu bei, daß der andere Klemmbacken eine sehr stabile Anordnung bildet.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines als Rohrschaft-instrument ausgebildeten bipolaren Faß-instrumentes mit zwei verschwenkbaren Klemmbacken;
- Figur 2:: eine perspektivische Explosionsdarstellung der beiden Klemmbacken und der Antriebselemente für die Klemmbacken;
- Figur 3:: eine Seitenansicht des Instrumentes der Figur 1 im Klemmbackenbereich mit den Klemmbacken in einer Faßposition;
- Figur 4:: eine Ansicht ähnlich Figur 3 mit den Klemmbacken in einer Schneidposition und
- Figur 5:: eine perspektivische Darstellung des mit einer Schneideinrichtung versehenen Klemmbackens ähnlich der Darstellung der Figur 2 bei einem abgewandelten Ausführungsbeispiel dieses Klemmbackens.

Das in der Zeichnung dargestellte chirurgische Instrument ist als Rohrschaftinstrument 1 ausgebildet mit einem länglichen, rohrförmigen Schaft 2 mit einem distalen Ende 3 und einem proximalen Ende 4.

Am proximalen Ende 4 ist der Schaft 2 mit einem Griffteil 5 verbunden, welches eine feststehende Branche 6 und eine schwenkbar mit dieser verbundene Branche 7 umfaßt, diese ist gelenkig mit einer Schub- und Zugstange 8 verbunden, die den Schaft 2 durchsetzt und durch Verschwenkung der Branche 7 relativ zum Schaft 2 in Längsrichtung vorschiebbar und zurückziehbar ist.

In den Schaft 2 ist an dessen distalem Ende 3 eine Halterung 9 eingesetzt, die an ihrem distalen Ende zwei parallel zueinander verlaufende, in distaler Richtung vorstehende Lagerarme 10 trägt, die einen Lagerzwischenraum 11 zwischen sich einschließen. In diesen ist ein Isolationskörper 12 aus einem elektrisch isolierenden Material eingesetzt, beispielsweise aus Keramik, der im wesentlichen U-förmig ausgebildet ist und die Lagerarme 10 an ihrer Innenseite abdeckt.

Die Zug- und Schubstange 8 durchsetzt sowohl die Halterung 9 als auch den Isolationskörper 12 und endet im Bereich des Lagerzwischenraumes 11.

Im Bereich ihrer freien Enden weisen die Lagerarme 10 miteinander fluchtende Aufnahmeöffnungen 13 für eine Lagerwelle 14 auf, die quer zur Längsrichtung der Schaftlängsachse des Instrumentes verläuft und den Lagerzwischenraum 11 an seinem distalen, offenen Ende durchsetzt.

Auf dieser Lagerwelle 14 sind zwei Klemmbacken 15, 16 nebeneinander und unabhängig voneinander verschwenkbar gelagert, deren proximale Enden 17, 18 in den Lagerzwischenraum 11 hineinragen. Jedes proximale Ende 17, 18 ist mit einem Lenker 19 bzw. 20 gelenkig verbunden, deren andere Enden ebenfalls gelenkig mit dem distalen Ende der Schub- und Zugstange 8 verbunden sind, so daß diese Lenker 19, 20 zusammen mit den proximalen Enden 17 bzw. 18 ein Viergelenk ausbilden, über welches beim Vorschieben und Zurückziehen der Schub- und Zugstange 8 die Klemmbacken 15 und 16 um die durch die Lagerwelle 14 gebildete Drehachse verschwenkbar sind: Beim Vorschieben der Schub- und Zugstange 8 werden die Klemmbacken auseinandergeschwenkt, d. h. geöffnet, beim Zurückziehen der Schub- und Zugstange 8 erfolgt ein Schließen der Klemmbacken 15, 16.

Die Klemmbacken 15 und 16 sind unterschiedlich ausgebildet. Einer der beiden Klemmbacken, im folgenden der untere Klemmbacken 16 genannt, ist dreiteilig ausgebildet, er umfaßt einen proximalen Abschnitt 21, der sich bis unmittelbar vor die Lagerwelle 14 erstreckt, einen mittleren Abschnitt 22, der sich im wesentlichen im Bereich der Lagerwelle 14 befindet, und einen distalen Abschnitt 23, dessen im wesentlichen ebene, dem anderen Klemmbacken 15 zugewandte Oberseite 24 eine Klemmfläche ausbildet, die zur Erhöhung der Griffigkeit mit Querrippen 25 versehen ist.

Der mittlere Abschnitt 22 besteht aus einem elektrisch isolierenden Material, beispielsweise aus Keramik, und verbindet den proximalen Abschnitt 21 mit dem distalen Abschnitt 23 unter elektrischer Isolierung dieser beiden Abschnitte, die aus Metall bestehen und damit elektrisch leitfähig sind. Der mittlere Abschnitt 22 liegt dabei flächig sowohl am distalen Abschnitt 23 als auch am proximalen Abschnitt 21 an, so daß im Überlappungsbereich eine Schichtstruktur entsteht, außerdem stützt sich der mittlere Abschnitt 22 über seine hintere Kante 26 und über eine vordere Anlagefläche 27 am proximalen Abschnitt 21 bzw. am distalen Abschnitt 23 ab, so daß auf diese Weise der proximale Abschnitt 21 mit dem distalen Abschnitt 23 elektrisch isolierend und starr verbunden ist.

Der mittlere Abschnitt 22 trägt seitlich eine einstükkig mit ihm ausgebildete Hülse 28, die Lageröffnungen 29 und 30 in den beiden Klemmbacken 15 und 16 durchsetzt und die in sich die Lagerwelle 14 aufnimmt, die dadurch gegenüber dem Klemmbacken 15 und dem proximalen Ende 18 des Klemmbackens 16 elektrisch isoliert ist. Die Lagerwelle 14 selbst besteht aus Metall und ist elektrisch leitend mit den Lagerarmen 10 der Halterung 9 verbunden, die ihrerseits in elektrisch leitender Verbindung mit dem Schaft 2 steht. Dieser trägt einen Elektroanschluß 31, mit dem eine elektrische Verbindung zu einer in der Zeichnung nicht dargestellten Hochfrequenzspannungsquelle herstellbar ist.

Die Lagerwelle 14 steht mit dem distalen Abschnitt 23 des Klemmbackens 16 in unmittelbarem Kontakt, so daß in diesem Bereich eine elektrisch leitende Verbindung zwischen dem distalen Abschnitt 23 und der Lagerwelle 14 entsteht. Dadurch kann der distale Abschnitt 23 des Klemmbackens 16 mit einem Pol der Hochfrequenzspannungsquelle verbunden werden.

Der andere Pol wird in aus der Zeichnung nicht ersichtlicher Weise mit der Schub- und Zugstange 8 verbunden, so daß auf diese Weise über die Lenker 19 und 20 sowohl der proximale Abschnitt 21 des Klemmbackens 16 als auch der gesamte Klemmbacken 15 mit diesem zweiten Pol der Hochfrequenzspannungsquelle verbunden sind.

Der mittlere Abschnitt 22, der im übrigen über einen in eine Bohrung 32 des proximalen Abschnittes 21 eingreifenden Stift 33 zusätzlich gegenüber dem proximalen Abschnitt 21 festgelegt ist, trennt den distalen Abschnitt 23 des Klemmbackens 16 elektrisch vom proximalen Abschnitt 21 des Klemmbackens 16, so daß die beiden Klemmbacken 15 und 16 elektrisch voneinander isoliert und jeweils mit einem Pol der Spannungsquelle verbunden sind.

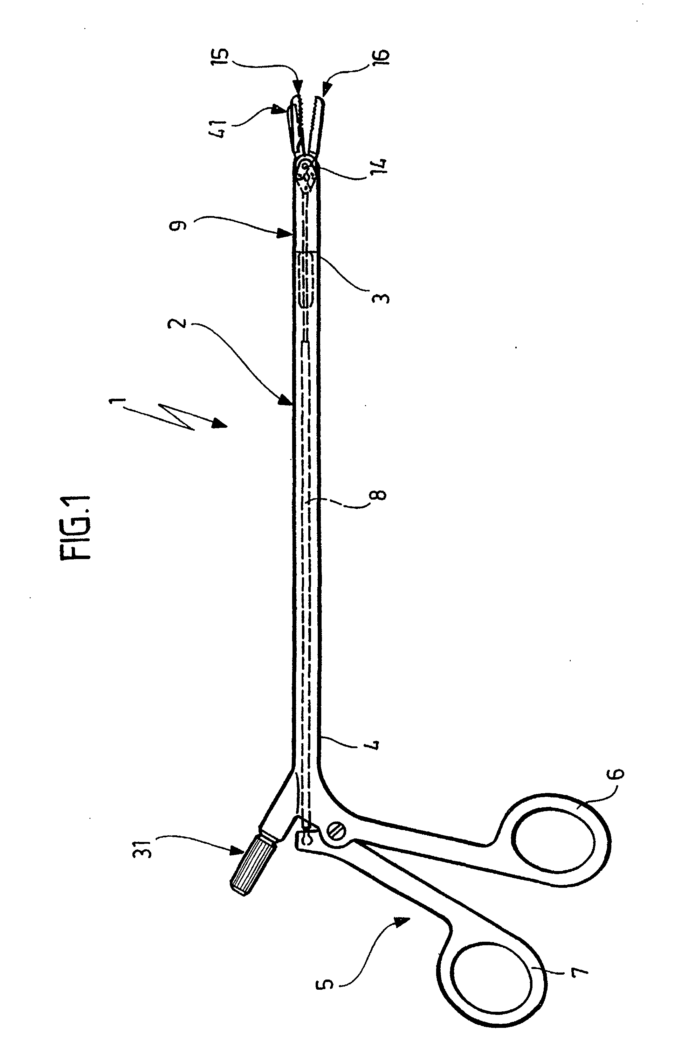

Der andere Klemmbacken, im folgenden als oberer Klemmbacken 15 bezeichnet, besteht insgesamt aus Metall und ist bei dem Ausführungsbeispiel der Figuren 1 bis 4 einstückig ausgebildet. Ein proximaler Abschnitt 34 ist dabei ähnlich ausgebildet wie der proximale Abschnitt 21 des unteren Klemmbackens 16, er geht über in einen distalen Abschnitt 35, der zwei parallel zueinander verlaufende, zwischen sich einen Längsschlitz 36 bildende Klemmelemente 37 aufweist, die gleich ausgebildet sind. Beide Klemmelemente 37 weisen auf ihrer dem unteren Klemmbacken 16 zugewandten Seite je eine Klemmfläche 38 auf, die der Klemmfläche 24 des unteren Klemmbackens 16 gegenübersteht und die ebenso wie diese mit Querrippen 39 versehen ist.

Beide Klemmelemente 37 sind mit dem proximalen Abschnitt 34 über bandförmige Stege 40 geringer Bauhöhe verbunden, die so flach ausgebildet sind, daß in diesem Bereich ein elastisches Verschwenken der armartigen Klemmelemente 37 möglich ist und zwar um eine Schwenkachse, die parallel zur Lagerwelle 14 verläuft.

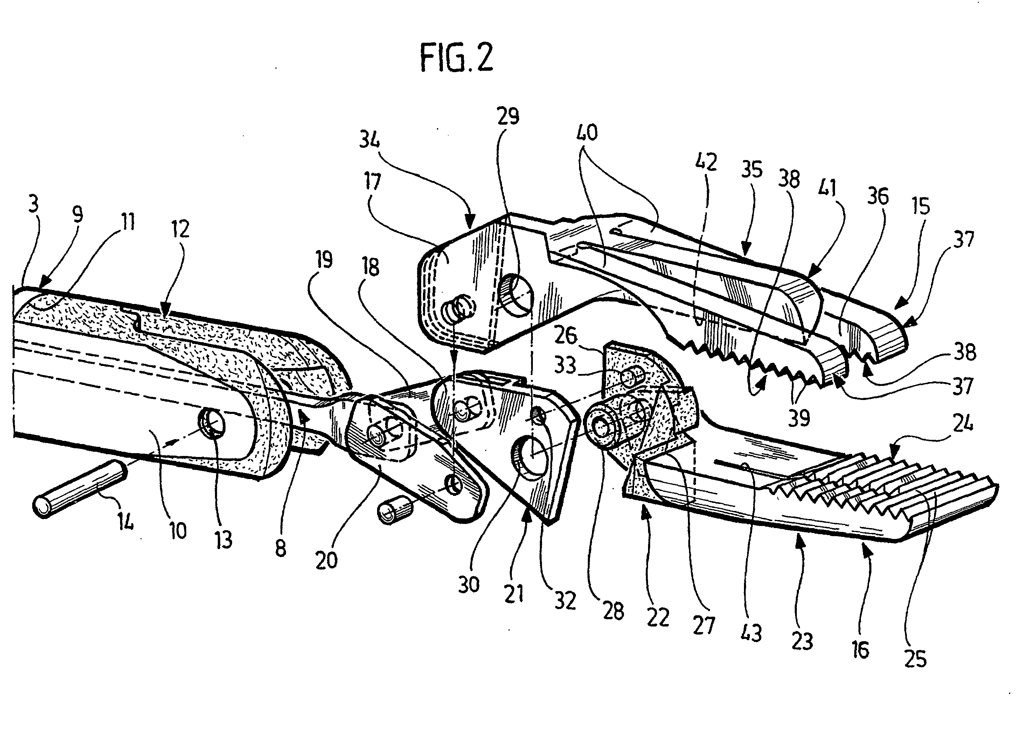

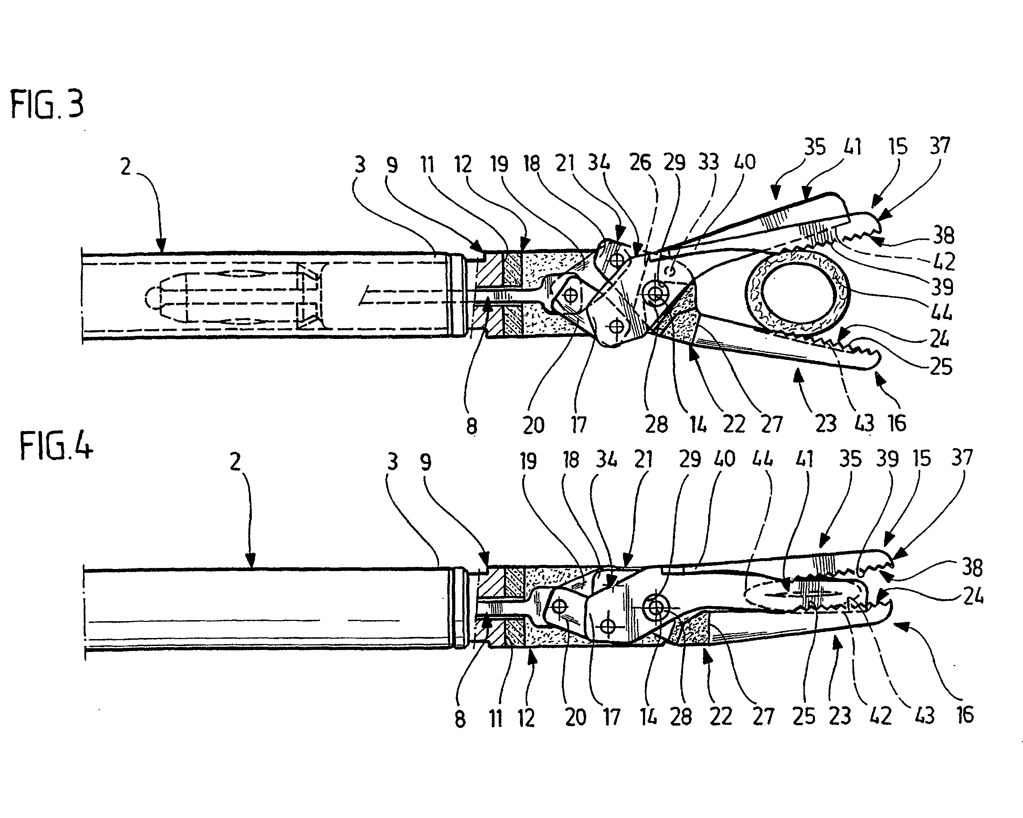

In den zwischen den beiden Klemmelementen 37 frei bleibenden Längsschlitz 36 ragt ein starr mit dem proximalen Abschnitt 34 verbundener Schneidkörper 41 hinein, dessen untere Kante als Schneide 42 ausgebildet ist.
Wenn die Klemmelemente 37 unverformt sind, wenn sie also nicht elastisch gegenüber dem proximalen Abschnitt 34 verschwenkt sind, befindet sich die Schneide 42 vollständig im Inneren des Längsschlitzes 36, sie steht also nicht nach unten über die Klemmflächen 38 der beiden Klemmelemente 37 vor (Darstellung der Figuren 2 und 3).

Wenn die Klemmelemente 37 dagegen elastisch nach oben verschwenkt werden, also in öffnungsrichtung der Klemmbacken, dann tritt diese Schneide 42 zwischen den Klemmflächen 38 nach unten hervor und kann wirksam werden.

Im unteren Klemmbacken 16 ist ebenfalls ein Längsschlitz 43 ausgebildet, der mit dem Längsschlitz 36 im oberen Klemmbacken 15 ausgerichtet und der an beiden Enden geschlossen ist, die Länge des Längsschlitzes 43 ist so bemessen, daß die Schneide 42 des Schneidkörpers 41 in den Längsschlitz 43 eintauchen kann.

Das in dieser Weise ausgebildete Instrument dient gleichermaßen als bipolares Faßinstrument und als Schneidinstrument.

Bei vorgeschobener Schub- und Zugstange 8 sind die Klemmbacken 15 und 16 zunächst auseinandergeschwenkt, die Faßzange befindet sich in der öffnungsstellung. In dieser Stellung kann zu behandelndes Gewebe, beispielsweise ein Gefäß 44, an den Klemmflächen 24 und 38 der Klemmbacken angelegt und ergriffen werden (Figur 3). Durch Anlegen einer Hochfrequenzspannung an die beiden Klemmbacken 15 und 16 ist auf diese Weise durch die Anlage des Gefäßes an den beiden Klemmbacken 15 und 16 eine Koagulation des Gefäßes 44 im Anlagebereich möglich, wie dies bei bipolaren Faßzangen an sich bekannt ist.

Diese Koagulation erfolgt bei einer Stellung der Klemmbacken 15, 16, bei denen diese gegenüber der Offenstellung in eine Zwischenstellung verschwenkt, aber noch nicht vollständig geschlossen sind. In dieser Zwischenstellung wird der Anpreßdruck des oberen Klemmbackens 15 an das Gefäß 44 noch so gering sein, daß die Klemmelemente 37 im Bereich der Stege 40 nicht elastisch verschwenkt werden, sondern unverformt bleiben, so daß der Schneidkörper 41 mit der Schneide 42 im Längsschlitz 36 verbleibt, die Schneide 42 also unwirksam ist (Figur 3).

Beim weiteren Schließen der Klemmbacken 15 und 16 durch Zurückziehen der Schub- und Zugstange 8 werden jedoch die Klemmbacken 15 und 16 kräftig gegeneinander gedrückt, und dies führt dazu, daß die Klemmelemente 37 im Bereich der dünnen, elastisch verbiegbaren Stege 40 verbogen werden, die Klemmelemente 37 verschwenken also entgegen der Schließstellung nach oben, und dadurch kann die Schneide 42 des Schneidkörpers 41 nach unten über die Klemmflächen 38 heraustreten, am Gefäß 44 zur Anlage kommen und dieses beim weiteren Schließen der Klemmbacken 15 und 16 durchtrennen, wobei der Schneidkörper 41 mit der Schneide 42 in den Längsschlitz 43 des Klemmbackens 16 eintaucht.

Mit derselben Schließbewegung der verschwenkbaren Branche 7 kann der Operateur also zunächst das zu behandelnde und zu durchtrennende Gewebe erfassen, dann durch Anlegen einer Hochfrequenzspannung koagulieren und schließlich durch weiteres Verschwenken der Branche 7 im koagulierten Bereich durchtrennen, es ist dazu nur ein Antriebsmechanismus notwendig, und der Operateur kann diesen Vorgang mit einem einzigen Hebel durchführen, den er entsprechend den Anforderungen so verschwenkt, daß nacheinander die beschriebenen Arbeitsstellungen eingenommen werden.

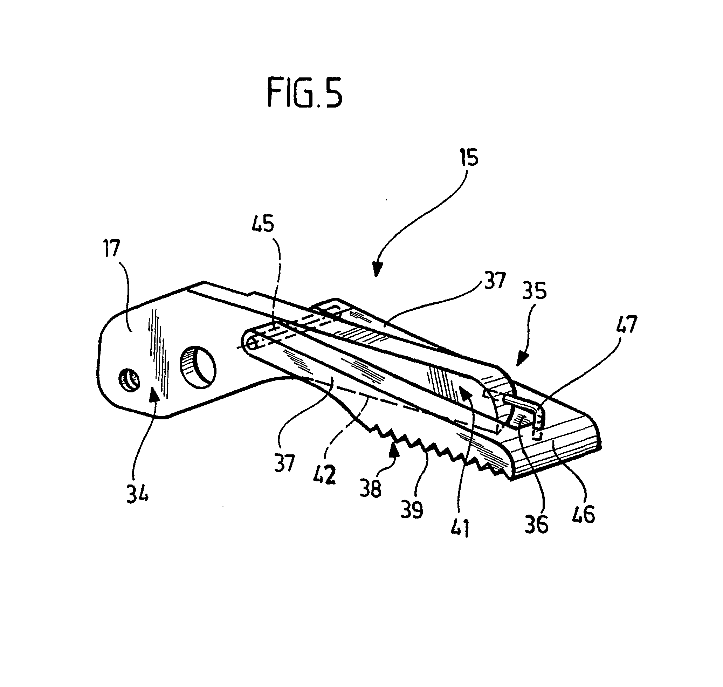

Das Ausführungsbeispiel der Figur 5 ist ähnlich aufgebaut wie das der Figuren 1 bis 4, einander entsprechende Teile tragen daher dieselben Bezugszeichen.

Ein Unterschied besteht lediglich im Bereich des oberen Klemmbackens 15, der bei dem Ausführungsbeispiel der Figur 5 zweiteilig ausgebildet ist. Die beiden Klemmelemente 37 sind bei diesem Ausführungsbeispiel nicht über elastische Stege 40 mit dem proximalen Abschnitt 34 des Klemmbackens 15 verbunden, sondern sie sind über einen Lagerstift 45 verschwenkbar am Schneidkörper 41 gelagert, und zwar im Übergangsbereich des Schneidkörpers 41 zum proximalen Abschnitt 34. An den distalen Enden sind bei diesem Ausführungsbeispiel die Klemmelemente 37 durch einen Quersteg 46 miteinander verbunden, so daß die beiden Klemmelemente 37 zusammen mit dem Quersteg 46 ein einheitliches Bauteil ausbilden.

Ein in der Zeichnung nur schematisch dargestelltes, einerseits mit dem Schneidkörper 41 und andererseits mit dem Quersteg 46 verbundenes Federelement 47 hält die Klemmelemente 37 in einer Stellung, in der die Klemmflächen 38 nach unten über die Schneide 42 des Schneidkörpers 41 hervorstehen, die Schneide 42 also vollständig im Längsschlitz 36 aufgenommen und daher unwirksam ist. Gegen die Wirkung dieses Federelementes 47 können die Klemmelemente 37 so elastisch verschwenkt werden, daß die Schneide 42 aus dem Längsschlitz 36 austritt und in der beschriebenen Weise wirksam wird.

Bei diesem Ausführungsbeispiel sind die Klemmelemente 37 über den Lagerstift 45 elektrisch leitend mit dem proximalen Abschnitt 34 verbunden, so daß auch in diesem Falle die Klemmflächen 38 mit einem Pol der Hochfrequenzspannungsquelle in Verbindung stehen.

## Patentansprüche

1. Bipolares Faßinstrument mit zwei gegeneinander bewegbaren, elektrisch voneinander isolierten und jeweils mit einem Pol einer elektrischen Hochfrequenzspannungsquelle verbindbaren Klemmbacken (15, 16), die jeweils zwei im Abstand zueinander angeordnete, zwischen sich einen Längsschlitz (36) ausbildende Klemmelemente (37) mit je einer Klemmfläche (38) aufweisen, und mit einer Schneideinrichtung, die ein in den Längsschlitzen der Klemmbacken (15, 16) verschiebbares Schneidelement mit einer Schneide (42) umfaßt, wobei das Schneidelement (41) im Längsschlitz (36) des einen Klemmbakkens (15) angeordnet ist, **dadurch gekennzeichnet, daß** die Schneide (42) im Längsschlitz (36) zwischen den Klemmelementen (37) dieses Klemmbackens (15) aufgenommen ist und nicht über dessen Klemmflächen (38) in Richtung auf den anderen Klemmbakken (16) übersteht, und daß die Klemmelemente (37) dieses Klemmbackens (15) relativ zu dem Schneidelement (41) entgegen der Schließbewegung der Klemmbacken (15, 16) elastisch so weit bewegbar sind, daß die Schneide (42) des Schneidelements (41) über die Klemmflächen (38) hervorsteht.

2. Instrument nach Anspruch 1, **dadurch gekennzeichnet, daß** sowohl das Schneidelement (41) als auch die Klemmelemente (37) einstückig mit dem Klemmbacken (15) ausgebildet sind und daß das Schneidelement (41) ein starres Teil des Klemmbackens (15) ist, während die Klemmelemente (37) über elastisch verformbare Bereiche (40) mit den übrigen Teilen (34) des Klemmbackens (15) verbunden sind.

3. Instrument nach Anspruch 2, **dadurch gekennzeichnet, daß** die elastisch verformbaren Bereiche (40) durch elastisch verbiegbare Stege mit geringer Höhe gebildet werden.

4. Instrument nach Anspruch 1, **dadurch gekennzeichnet, daß** der Klemmbacken (15) zweiteilig ausgebildet ist mit einem starren ersten Teil, welches das Schneidelement (41) und die Lagerung des Klemmbakkens (15) am Faßinstrument umfaßt, und mit einem zweiten Teil, welches die Klemmelemente (37) umfaßt und welches am ersten Teil elastisch bewegbar gelagert ist.

5. Instrument nach Anspruch 4, **dadurch gekennzeichnet, daß** die Klemmelemente (37) elastisch verschwenkbar an dem ersten Teil gelagert sind.

6. Instrument nach Anspruch 5, **dadurch gekennzeichnet, daß** das zweite Teil U-förmig ausgebildet ist mit zwei parallelen, die Klemmelemente (37) bildenden Schenkeln und einem diese verbindenden, den Längsschlitz (36) zum distalen Ende des Klemmbakkens (15) hin abschließenden Steg (46), und daß das zweite Teil an den freien Enden der Schenkel drehbar an dem ersten Teil gelagert ist.

7. Instrument nach einem der voranstehenden Ansprüche, **dadurch gekennzeichnet, daß** der Längsschlitz (43) in dem anderen Klemmbacken (16), der keine Schneideinrichtung aufweist, an beiden Enden geschlossen ist.

## Claims

1. A bipolar gripping instrument comprising two clamping jaws (15, 16) which are movable relative to one another, are electrically insulated from one another and are each connectable to a pole of an electrical high-frequency voltage source, and which each have two mutually spaced clamping members (37) forming a longitudinal slot (36) between them and each having a clamping surface (38), and a cutting device which comprises a cutting member having a cutting edge (42) and being displaceable in the longitudinal slots of the clamping jaws (15, 16), wherein the cutting member (41) is arranged in the longitudinal slot (36) of one clamping jaw (15), **characterised in that** the cutting edge (42) is received in the longitudinal slot (36) between the clamping members (37) of this clamping jaw (15) and does not project beyond its clamping surfaces (38) towards the other clamping jaw (16), and **in that** the clamping members (37) of this clamping jaw (15) are resiliently movable relative to the cutting member (41) counter to the closing movement of the clamping jaws (15, 16) to such an extent that the cutting edge (42) of the cutting member (41) projects beyond the clamping surfaces (38).

2. An instrument according to claim 1, **characterised in that** both the cutting member (41) and the clamping members (37) are formed in one piece with the clamping jaw (15) and **in that** the cutting member (41) is a rigid part of the clamping jaw (15), whereas the clamping members (37) are connected to the other parts (34) of the clamping jaw (15) via elastically deformable regions (40).

3. An instrument according to claim 2, **characterised in that** the elastically deformable regions (40) are formed by elastically deformable bridge pieces of low thickness.

4. An instrument according to claim 1, **characterised in that** the clamping jaw (15) is formed in two parts by a rigid first part, which comprises the cutting member (41) and the mounting of the clamping jaw (15), and a second part which comprises the clamping members (37) and is mounted on the first part so as to be resiliently movable.

5. An instrument according to claim 4, **characterised in that** the clamping members (37) are mounted on the first part so as to be resiliently pivotable.

6. An instrument according to claim 5, **characterised in that** the second part is U-shaped and has two parallel arms forming the clamping members (37) and a bridge piece (46) connecting the latter and closing the longitudinal slot (36) in the direction of the distal end of the clamping jaw (15), and **in that** the second part is rotatably mounted on the first part at the free ends of the arms.

7. An instrument according to any one of the preceding claims, **characterised in that** the longitudinal slot (43) in the other clamping jaw (16), which is not provided with a cutting device, is closed at both ends.

## Revendications

1. Instrument de saisie bipolaire comportant deux mâchoires de serrage (15, 16) déplaçables l'une par rapport à l'autre, électriquement isolées l'une par rapport à l'autre et susceptibles d'être reliées chacune avec un pôle d'une source de tension électrique à haute fréquence, lesquelles présentent chacune deux éléments de serrage (37) comportant chacun une surface de serrage (38), agencés à distance l'un de l'autre et formant entre eux une fente longitudinale (36), et comportant un système de coupe qui comprend un élément de coupe avec un tranchant (42), déplaçable dans les fentes longitudinales des mâchoires de serrage (15,16), l'élément de coupe (41) étant agencé dans la fente longitudinale (36) de l'une des mâchoires de serrage (15), **caractérisé en ce que** le tranchant (42) est reçu dans la fente longitudinale (36) entre les éléments de serrage (37) de cette mâchoire de serrage (15) et ne fait pas saillie au-delà de ses surfaces de serrage (38) en direction de l'autre mâchoire de serrage (16), et **en ce que** les éléments de serrage (37) de cette mâchoire de serrage (15) peuvent être déplacés de manière élastique par rapport à l'élément de coupe (41) à l'encontre du mouvement des mâchoires de serrage (15, 16) aussi loin que le tranchant (42) de l'élément de coupe (41) fait saillie au-delà des surfaces de serrage (38).

2. Instrument selon la revendication 1, **caractérisé en ce que** tant l'élément de coupe (41) que les éléments de serrage (37) sont réalisés d'un seul tenant avec la mâchoire de serrage (15) et **en ce que** l'élément de coupe (41) est une partie rigide de la mâchoire de serrage (15) tandis que les éléments de serrage (37) sont reliés aux autres parties (34) de la mâchoire de serrage (15) via des régions (40) élastiquement déformables.

3. Instrument selon la revendication 2, **caractérisé en ce que** les régions (40) élastiquement déformables sont formées par des traverses de faible hauteur élastiquement flexibles.

4. Instrument selon la revendication 1, **caractérisé en ce que** les mâchoires de serrage (15) sont réalisées en deux parties avec une première partie rigide qui comprend l'élément de coupe (41) et la monture de la mâchoire de serrage (15) sur l'instrument de saisie, et avec une deuxième partie qui comprend les éléments de serrage (37) et qui est montée de manière élastiquement déplaçable sur la première partie.

5. Instrument selon la revendication 4, **caractérisé en ce que** les éléments de serrage (37) sont montés de manière élastiquement pivotante sur la première partie.

6. Instrument selon la revendication 5, **caractérisé en ce que** la deuxième partie est réalisée en forme de U avec deux branches parallèles formant les éléments de serrage (37) et avec une traverse (46) reliant les branches et fermant la fente longitudinale (36) vers l'extrémité distale de la mâchoire de serrage (15), et **en ce que** la deuxième partie est montée rotative sur la première partie aux extrémités libres des branches.

7. Instrument selon l'une des revendications précédentes, **caractérisé en ce que** la fente longitudinale (43) est fermée aux deux extrémités dans l'autre mâchoire de serrage (16) qui ne présente pas de système de coupe.
